# EUROPEAN PATENT SPECIFICATION

(11) **EP 3 272 373 B1**
(45) Date of publication and mention of the grant of the patent: **14.08.2019**
(21) Application number: 16382674.6
(22) Date of filing: 30.12.2016
(51) Int. Cl.: A61M 1/02, A61B 5/15, A61B 5/157, A61J 1/14

(54) **TRANSFUSION SAFETY DEVICE**
TRANSFUSIONSSICHERHEITSVORRICHTUNG
DISPOSITIF DE SÉCURITÉ DE TRANSFUSION

(30) Priority: 21.07.2016 ES 201630992
(43) Date of publication of application: 24.01.2018
(73) Proprietor: Proposit Bio, S.L., 28232 Las Rozas(Madrid) (ES); Parrado González, Carmelo, 28232 Madrid (ES); Hurtado Mondéjar, Maria del Carmen, 28232 Las Rozas(Madrid) (ES)
(72) Inventor: BUENO CABRERA, José Luis, 28016 Madrid (ES)
(74) Representative: Pons Ariño, Angel

(56) References cited:
- US-A1- 2005 148 033
- US-A1- 2013 060 228
- US-A1- 2014 363 828

## Description

### OBJECT OF THE INVENTION

The present invention falls within the technical field of devices for taking blood samples, specifically in that of those intended to carry out in vivo measurements of blood characteristics, as well as in that of means for controlling the flow of agents towards the body or to dispense the agents to be introduced into the body, and in that of chemical analysis of biological material and immunological research and analysis in which blood groups or types are involved, and in particular it relates to a device for checking the blood group of the blood contained in a transfusion unit and the blood of a transfusion recipient and determining the compatibility thereof before carrying out a transfusion.

### BACKGROUND OF THE INVENTION

Blood transfusion is a very common medical procedure; it is estimated that 2 million blood components are transfused in Spain every year. The majority of said transfused components are packed red blood cells, which are the amount of red blood cells that are obtained from a blood donation once they are separated from the rest of the blood components, and which are generally used to increase the red cell mass of the recipient of the transfusion.

The main risks associated with transfusions are those derived from pulmonary complications that may arise, as well as due to errors in the administration of an unsuitable blood component, which takes place when packed red blood cells of a blood group, also referred to as ABO group, that is incompatible with that of the recipient are transfused, and possibly even leading to death. In fact, it is considered that today in Spain, errors in the administration of packed red blood cells that are incompatible are the leading cause of deaths related to transfusion.

These types of incident take place due to a series of possible errors, which may be classified into three main groups. Firstly, there are errors that take place when the sample is taken from the recipient in order to carry out a study before the transfusion, such as when the healthcare professional that has extracted the sample incorrectly labels the tube where the blood is extracted with the information of another individual. Secondly, there are errors in the labelling of the packed red blood cells themselves, due to errors made by the staff at blood banks when labelling the bags with an individual's name that is different to the real name. Lastly, there are errors derived from the incorrect administration of the component at the time when the transfusion is carried out, in which the nursing staff do not correctly identify the individual to which the transfusion is to be applied, introducing incompatible packed red blood cells into their organism, which were intended for another individual.

Lastly, there is the risk that the packed red blood cells contain red blood cells of a different ABO group than that which appears on the label thereof; or even, that the sample tube that is usually used to identify the ABO group of the bag is different to the ABO group of the red blood cells contained in said bag, as a result of incorrect handling during the production of the component, or even due to malicious tampering.

Various devices, and the corresponding associated methods thereof, which are aimed at preventing these errors are known in the state of the art. Said prevention methods are usually based on placing identification devices, such as bracelets, on which appears the basic information about the individual to which the packed red blood cells are to be transfused. These methods require strict protocols to be followed, for which all the staff involved in the transfusion process must be trained and it must be ensured that it is strictly followed. The operation of a computer system is also often necessary, which is made up of both the software and the computer devices themselves, in order to carry out the adequate monitoring and control of the entire process. In any case, the efficiency of these systems or methods is subject to the staff involved in the transfusion process strictly following of the protocol established.

Firstly, these methods entail a considerable increase in the cost of the transfusion process, which is currently quantified at 5-7 euros per transfusion carried out, and they are not infallible due to factors such as the lack of staff training or due to needs caused by emergency situations, meaning that the established protocol is not strictly followed, and as a result, incorrect transfusions are carried out despite the fact that there is a pre-established transfusion safety method. Currently, a solution that prevents the possible boycott or accidental error is not known of the market, since the order for carrying out the connection between the bag of red blood cells to be transfused and the channel of the recipient is always in the hands of the healthcare professionals.

Furthermore, the systems available are not capable of preventing an incident due to ABO incompatibility due to an error in the labelling of the packed red blood cells, or when there is a discrepancy between the blood inside the bag and the sample tube used to determine the ABO group of the bag.

For instance, the patent document with publication number US2013060228 discloses a secure system for the perfusion of a body liquid, enabling a final control of compatibility of a treatment with the patient and/or the medical situation previously diagnosed by a doctor. To this end, the system comprises: a fluidic circuit of a perfusion comprising a perfusion catheter, a perfusion tubing and a container for the determined perfusion product to be perfused to a patient; a means for taking a body liquid sample from a patient; a sampling container provided with a means for fluidic connection to the means for taking the sample; a means of analysis and comparison of the body liquid taken and a sample of the determined product; a means for fluidic connection between the analysis means and the sampling container; and a means for controlling the flow of the perfusion product in the tubing, the analysis and comparison means transmitting information for comparing the body liquid taken and the determined production sample to the flow control means.

The process disclosed by requires from the nursing staff to separate a sampling container from the main container of the transfusion bag and plugging it in the upper-right connector of flow control means for the analysis means to analyse a sample of the blood corresponding to the blood bag.

Patent publication document US2005148033 relates to a method and device for testing a blood product and a patient's blood immediately prior to transfusing the blood product to the patient. The device comprises a testing chamber within which the blood product and the patient blood are drawn and tested for compatibility. A first end of the testing chamber is coupled with an infusion needle in communication with the patient. A second end of the testing chamber is in fluid communication with the container housing the blood product. In an embodiment, blood product and patient blood are drawn into the testing chamber. One of a number of tests for determining blood compatibility is performed in the testing chamber. If the blood product and patient blood are compatible, blood product is delivered to the patient through a transfusion line bypassing the testing chamber.

Based on the foregoing, the need for a transfusion safety system that prevents at least the incompatibility errors in the blood group, referred to hereinafter as ABO group, which is not subject to following a method or protocol, and which is physically capable of preventing a transfusion when there is an incompatibility between the ABO group of the individual and the ABO group of the bag is deduced.

### DESCRIPTION OF THE INVENTION

The invention is defined in the appended claims.

The object of the invention consists of a device for ensuring the control transfusion practise of packed red blood cell units, referred to hereinafter by its acronym PRBCs, via the use of an immunochemical system for detecting the ABO group in the PRBCs and in the individuals blood, and an automatic control device capable of identifying the ABO result of the immunochemical test and that additionally integrates checking the compatibility between the blood component and the blood of the individual, causing the automatic release of a flow regulation device that enables the transfusion or infusion of PRBCs to the individual to start, only in the case of compatibility of the ABO group between the PRBCs and the blood of the individual. In this way, adverse reactions in the individual linked to a possible incompatibility between the ABO groups are prevented.

To do so, the transfusion safety device incorporates, in a traditional PRBC infusion system, at least two ABO blood group checking devices, at least one control unit, which is preferably a PDA-type handheld computer unit, which is capable of reading and interpreting the results shown by the checking device, and a PRBC flow regulation device, preferably a solenoid-type valve, inserted in the duct of the infusion system to control the passage of said PRBCs therethrough depending on the commands received from the control unit, which carries out a computerised compatibility test between the ABO result detected in each of the checking devices.

The blood group checking devices, based on an antibody-antigen type reaction, are filled by gravity or capillarity and barely requires the input of sample drops through a small capillary. The incorporation of transfusion safety devices with at least one first device, intended to carry out the check of the ABO blood group corresponding to the PRBCs to be transfused, and a second device to check the ABO blood group of the individual receiving said transfusion is envisaged.

The antibody-antigen reaction system gives a result that is shown on the checking device itself, the display and interpretation of which may be carried out visually by the user as well as by the computerised reading unit.

The casing in which both checking devices are housed is designed to be physically coupled at the time of reading, such that the possibility of errors derived from a double reading by the control unit is prevented, i.e. that said control unit reads the same chamber twice by mistake. In turn, the infusion line that enables the PRBCs to pass to the individual remains blocked by the regulation device, which may only be released when the control unit that reads both checking chambers determines that there is a compatible result between the ABO group detected in the checking device for the PRBCs and the checking device for the blood of the individual.

The control unit is preferably equipped with an application that is firstly responsible for reading the results obtained by the ABO blood group checking devices, and secondly, interpreting said results based on an algorithm that includes the possible compatibility and incompatibility combinations between the ABO blood groups. As a result of said interpretation, the control unit sends a signal depending on the result of the test to the flow regulation device in order to enable or prevent the passage of the PRBCs to the individual.

Unlike other equipment and methods known in the state of the art, the device is not based on direct agglutination that compares blood samples (a cross-over trial between individual and PRBC product), but rather the use of a quick test to determine the applicable ABO blood group regardless of the individual and product in order to be used before the transfusion. The final determination of the compatibility between both results is carried out with an external control unit.

The main advantages provided by the device consist in that it physically prevents the transfusion of PRBCs to an individual when there is an incompatibility in the ABO group; being effective even when the staff involved in the transfusion have not been trained for it. Another important advantage consists in the speed of the checking process, since it may be carried out in less than 1 minute using a low volume of PRBCs and no more than 1 ml of the blood of the individual. Moreover, the device is capable of detecting an ABO incompatibility even when there is an error in the labelling of the PRBC bag, or when the content of the sample tube used to determine the ABO group of the bag is different to the ABO group of the blood contained in the PRBC bag.

Additionally, the device is advantageous because it is not necessary to test the ABO group of the individual again for successive transfusions, since the result obtained the first time is visible in the checking device thereof, entailing an additional time saving. Lastly, the device is disposable and enables an economic implementation of a global transfusion safety protocol at the hospital level, since it is estimated that it will only make each transfusion procedure €3-5 more expensive.

In turn, in an alternative embodiment, the checking device of the product to be transfused may be pre-connected and checked in the PRBC preparation laboratory, which is usually a transfusion centre, the reading thereof before the transfusion being sufficient.

The device may be an alternative to other transfusion safety systems or may be complementary thereto. It even enables double checking with the current systems for identifying the bracelets of individuals, if a QR code is added, which is preferably the dynamic type, to the product checking device, the configuration of which contains the identification number of the individual, which generally appears on an identifying bracelet and the reading of which before the transfusion confirms that the allocation of the product is unequivocal for the individual who receives the transfusion.

However, and for the specific case of urgent transfusions, catastrophes or other extraordinary circumstances, it is envisaged that the device incorporates an alternative that enables the transfusion safety protocol to be overridden under the responsibility of the healthcare professional in charge of supervising the process.

Furthermore, and given that each unit of PRBCs transfused is unambiguously linked to an ABO group checking test carried out in the checking device, this test may be carried out at the individuals bedside or beforehand, and even in the transfusion centre itself when the PRBCs are produced.

In this way, each PRBC produced comprises a checking device where the ABO group of the PRBC is determined, which is used at the time of the transfer to carry out the checking compared to the ABO group of the individual. This device enables the checking process of the compatibility between a PRBC checking device that has been carried out beforehand and a checking device for the ABO of the individual, which in turn has been carried out once, and is valid for successive PRBC transfusions, to be sped up.

### DESCRIPTION OF THE DRAWINGS

In order to complement the description being made and with the object of helping to better understand the characteristics of the invention, in accordance with a preferred practical embodiment thereof, said description is accompanied, as an integral part thereof, by a set of drawings where, in an illustrative and non-limiting manner, the following has been represented:
Figure 1 shows a schematic view of the transfusion safety device, wherein the main constituent elements may be seen.
Figure 2 shows a detailed view of one of the blood group checking devices, wherein it may be seen how it couples to the other device before the results are read.

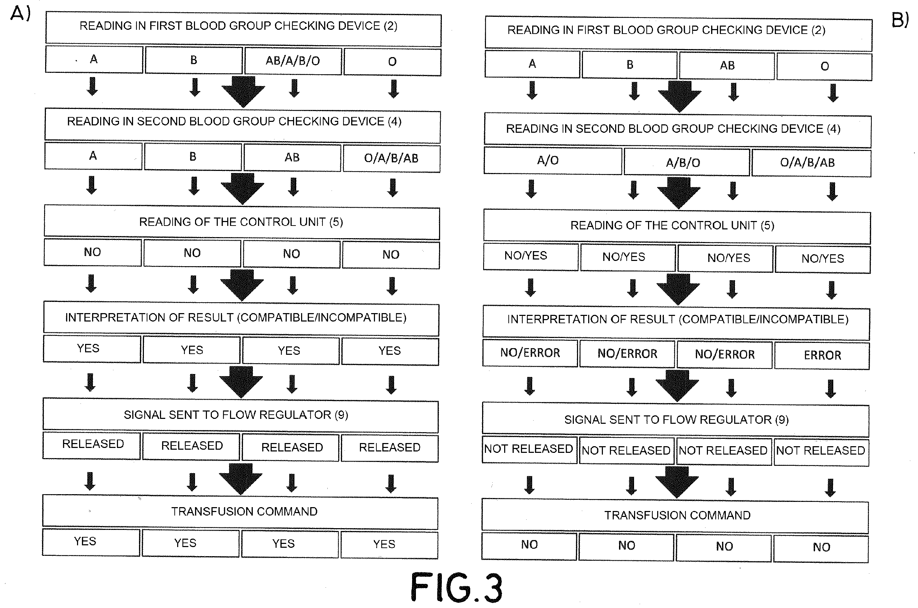

Figure 3 shows a schematic view of the response algorithm introduced in the control unit, wherein column A corresponds to the possible situations in which the transfusion is allowed and column B to those in which it is not allowed.

### PREFERRED EMBODIMENT OF THE INVENTION

What follows is a detailed explanation, with the help of the aforementioned figures, of the preferred exemplary embodiment of the object of the present invention.

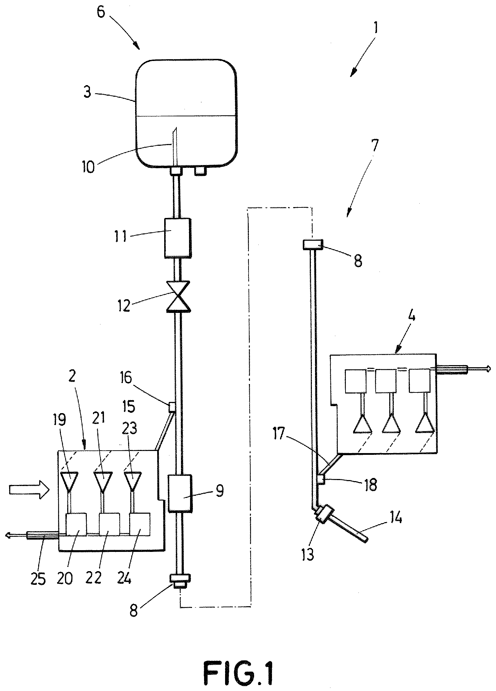

The transfusion safety device that is described, shown schematically in figure 1, is made up of a blood transfusion unit (1), to which a first blood group checking device (2) is linked via connections, which is intended to identify the blood group of packed red blood cells, which is also referred to using its acronym PRBCs, contained in a bag (3), and a second blood group checking device (4) intended to specify the blood group of an individual who is to receive the transfusion. This second device may be additionally be used for any type of intravenous infusion, which is also referred to as venoclysis, which is different to blood transfusions.

A control unit (5) reads the results obtained in the first (2) and second (4) blood group checking devices to establish the compatibility or incompatibility of both groups, depending on a previously introduced algorithm, and enable or prevent the passage of a PRBC flow from the bag (3) to the individual. In the preferred embodiment described herein, said control unit (5) is a PDA-type electronic handheld device provided with a result reading application.

It is provided that the first (2) and second (4) checking devices have a geometry that is complementary to each other, which enables them to be unequivocally coupled when the results are read, thus preventing errors derived from double readings or similar mistakes.

The blood transfusion unit (1) in turn comprises a bag line (6) and an individual's line (7), which are linked to each other when the transfusion is to be carried out via a connection (8). The bag line (6) additionally incorporates, in a position before the connection (8), a flow regulator (9) that may be actuated by the control unit (5).

In the preferred embodiment described herein, the connection (8) is of the male-female luer type, each of the respective portions of the connection being arranged at the free end of each one of the lines (6, 7) whilst the flow regulator (9) comprises a magnetic valve that operates like a two-position relay or solenoid valve, normally open or normally closed, to regulate the passage of blood therethrough depending on the commands received from the control unit (5).

The bag line (6) further comprises, at the opposite end to which the individual's line (7) is linked, a punch (10) intended to be inserted in the bag (3) in order to extract and channel the PRBCs contained in the inside thereof. The incorporation of a macroaggregate filter (11) is additionally provided, in a position behind the punch (10), in order to retain clots, cell debris and other waste present in the PRBC contained in the bag (3), and prevent the introduction thereof in the recipient. A roller clamp (12) regulates the flow rate of the PRBCs through the bag line (6).

Furthermore, the individual's line (7) has, at the opposite end to which the bag line (6) is linked, a luer-type connection point (13), intended to be inserted in a channel made in a vein on the individual receiving the transfusion. In order to prevent errors, it is provided that the individual's line (7) incorporates the needle itself of the channel (14) to be made in the individual. The flow regulator (9) that links the bag line (6) to the individual's line (7) thus controls the passage of a PRBC flow from the bag (3) to the channel (14).

A first bypass (15) takes a sample of the PRBCs that circulate through the bag line (6) and take it to the first blood group checking device (2) to identify the blood group corresponding to said PRBCs. The interposition of a first check valve (16) is provided in the first bypass (15) in order to prevent the return flow of said PRBC sample towards the bag line (6).

A second bypass (17) takes a sample of the blood of the individual from the channel (14) and takes it to the second blood group checking device (4) to establish the blood group corresponding to the patient. The interposition of a second check valve (18) is provided in the second bypass (17) in order to prevent the return flow of said blood sample towards the channel (14).

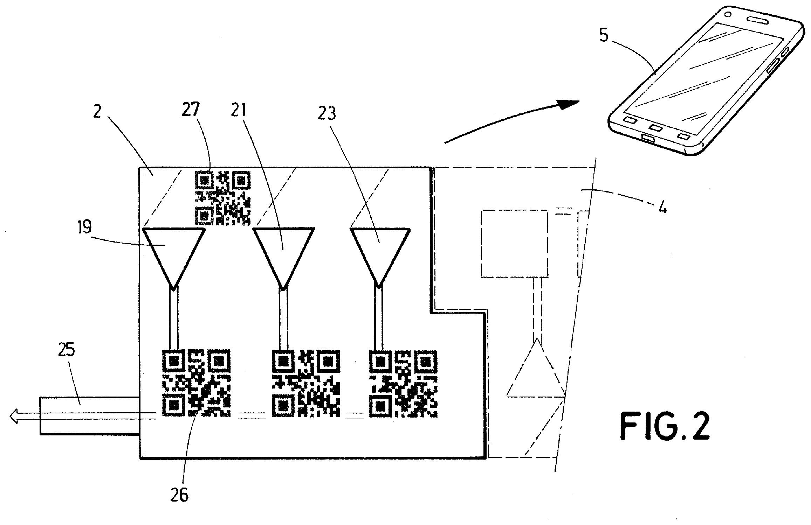

The determination of the blood group carried out both in the first (2) and the second (4) blood checking device is based on an antibody-antigen type agglutination reaction. Each one of said devices (2, 4), schematically shown in figure 2, comprises a first reaction chamber (19) that contains an anti-A reagent, linked to a first reading chamber (20), and a second reaction chamber (21) that contains an anti-B reagent, linked to a second reading chamber (22). A third reaction chamber (23) without antibodies, linked to a third reading chamber (24), operates as a control in order to ensure that the input of the sample has not been blocked due to causes different to the agglutination reaction.

Given that both the first (15) and second (17) bypasses have a very small, capillary-type diameter, the incorporation of a piston pump vacuum generating device (25) at the inlet of both blood group checking devices (2, 4) in order to facilitate the filling thereof with the respective samples is provided.

Each reading chamber (20,22,24) incorporates a visual information element (26), which may be a previously printed two-dimensional code or QR code, which contains information regarding the ABO blood group and the control of both the bag and the recipient. This visual information element (26) may or may not be legible by the control unit (5) depending on the agglutination reaction produced in the reading chambers (20,22), except in the case in which the visual information element (26) corresponds to the third reading chamber (24), which is always legible, since it acts as a control test.

The incorporation of a dynamic QR code (27) that contains information regarding the individual intended to receive the PRBCs contained in the bag (3) into the first blood group checking device (2) is additionally considered. Said information is generally contained in an identifying bracelet fastened to the individual, and the reading thereof by the control unit (5) in a stage prior to the transfusion confirms that the allocation of the product is unequivocal for the individual receiving the transfusion, further enabling the PRBC transfusion procedure to be tracked.

The control unit (5), which is a PDA in this preferred embodiment as stated above, is equipped with algorithms to determine the compatibility or incompatibility between blood groups. Said control unit (5) carries out a prior reading by scanning the visual information element (26) in order to be able to carry out said determination.

The PRBC or blood sample, depending on the device (2, 4) concerned, enters by capillarity in the first (19) and second (21) reaction chambers, and reacts with one, both or none of the reagents contained in said chambers. The reaction produces an agglutination that prevents the sample from flowing towards the first (20) or second (22) reading chamber. In the event that said agglutination takes place, the visual information element (26) is legible.

In the event that compatibility is established between both blood groups, the control unit (5) sends a command to the flow regulator (9) to enable the passage therethrough of the flow of PRBCs from the bag (3) towards the connection point (13) with the channel (14) of the individual, whilst if it is determined that the groups are incompatible, it does not send any signal, the flow regulator (9) remaining in its closed position.

In an alternative embodiment of the invention, the bags (3) containing PRBCs are prepared with the bag line (6) incorporated, which makes the punch (10) unnecessary. Therefore, the transfusion centres send the bags (3) with the bag line (6) thereof, which prevents a user from changing a bag (3) with the punch (10) to another that is incompatible, after the flow regulator (9) has been opened.

The transfusion method that uses the device described comprises the following sequence of actions:
- reading by the control unit (5) of the dynamic QR code (27) fastened in the first blood group checking device (2),
- connection of the bag line (6) to the bag (3) containing PRBCs through the punch (11),
- connection of the individual's line (7) to the channel (14) through the connection point (13),
- joining the bag line (6) with the individual's line (7) through the connection (8),
- inlet of a PRBC sample from the bag (3) to the first blood group checking device (2) through the first bypass (15),
- inlet of a blood sample from the channel (14) to the second blood group checking device (4) through the second bypass (17),
- linking the first (2) and second (4) blood group checking device to each other,
- simultaneous reading, by the control unit (5), of the results shown in the respective reading chambers (20,22,24) of the checking devices (2,4) by scanning the visual information elements (26), which in this case are QR codes, which are visible after the agglutination reactions produced in the respective reaction chambers (19,21,23).
- determining, by means of the control unit (5) of the compatibility or incompatibility between PRBCs and blood, and
- in the case of compatibility: sending a command from the control unit (5) to the flow regulator (9) for the opening thereof, thus enabling the flow of PRBCs to pass from the bag (3) towards the channel (14), or
- in the case of incompatibility: not sending any command to the flow regulator (9), which remains in a closed position preventing the flow of PRBCs to pass through.

By way of exemplary operation, in the first reading chamber (20), linked to the first reaction chamber (19), the corresponding visual information element (26) thereof is only legible if there have been prior agglutination between the red blood cells of the sample and the anti-A reagent contained in the said first reaction chamber (19). If this is not the case, if agglutination does not take place, the blood flows towards the first reading chamber (20) and prevents the reading.

If none of the visual information elements (26) corresponding to the first (20) and second (22) reading chambers can be read, the algorithm introduced in the control unit (5) interprets this as group O. In the case where agglutination takes place in both the first (19) and the second (21) reaction chambers, both visual information elements (26) are read, which is interpreted by the algorithm as group AB. In the event that the blood does not reach the third reading chamber (24), the printed code of which corresponds to the control, it must be considered that an error has occurred in the device, given that the absence of antibodies does not justify an agglutination and may be due to incorrect filling of the reaction chambers (19,21,23), which requires a new group checking.

## Claims

1. A transfusion safety device, intended to carry out a check prior to a transfusion of the blood group of packed red blood cells (PRBCs), contained in a bag (3) and the blood group of the blood of an individual receiving a transfusion and determine the compatibility thereof to enable or prevent said transfusion, the device comprising:
- a blood transfusion unit (1) which in turn comprises:
- a bag line (6), intended to be linked to the bag (3) containing the PRBCs,
- an individual's line (7), intended to be linked to an individual receiving the transfusion,
- a connection (8) to link the bag line (6) to the recipient's line (7), and
- a flow regulator (9) arranged in the bag line (6), which controls the passage of the flow of PRBCs circulating between the bag line (6) and the individual's line (7),
- a first blood group checking device (2) intended to establish the blood group of the PRBCs contained in the bag (3),
- a second blood group checking device (4) intended to establish the blood group of the individual receiving the transfusion, located at the end of a second bypass (17) that starts from the individual's line (7), and
- a control unit (5), intended to simultaneously read the results obtained in the first (2) and second (4) blood group checking devices, to establish the compatibility or incompatibility of both blood groups based on the reading of the results obtained in the first (2) and second (4) blood group checking devices, and to actuate the flow regulator (9) in order to enable or prevent the passage therethrough of the flow of PRBCs,
being the device **characterised in that**:
- the first blood group checking device (2) is located at the end of a first bypass (15) that starts from the bag line (6), and **in that**
- each one of the first (2) and second (4) blood group checking devices further comprises:
- a first reaction chamber (19), wherein an anti-A reagent for the agglutination of A group antibodies is housed, linked to a first reading chamber (20),
- a second reaction chamber (21), wherein an anti-B reagent for the agglutination of B group antibodies is housed, linked to a second reading chamber (22),
- a third reaction chamber (23), linked to a third reading chamber (24) for a control, and
- a plurality of visual information elements (26) linked to each one of the reading chambers (20,22,24).

2. The transfusion safety device according to claim 1, **characterised in that** the bag line (6) further comprises:
- a punch (10) intended to be introduced in the bag (3) to extract the PRBCs contained in the inside thereof, and
- a macroaggregate filter (11) to retain waste present in the PRBCs contained in the bag (3).

3. The transfusion safety device according to claim 1, **characterised in that** the individual's line (7) further comprises a connection point (13) intended to be inserted in a channel (14) made in a vein of the recipient.

4. The transfusion safety device according to claim 1, **characterised in that** the plurality of visual information elements (26) is a plurality of QR codes.

5. The transfusion safety device according to claim 1, **characterised in that** the first (2) and second (4) blood group checking devices have a geometry that is complementary to each other in order to facilitate the linking thereof prior to the reading of their results by the control unit (5).

6. The transfusion safety device according to claim 1, **characterised in that** the first bypass (15) incorporates a first check valve (16) to prevent a return flow of said PRBC sample.

7. The transfusion safety device according to claim 1, **characterised in that** the second bypass (17) incorporates a second check valve (18) to prevent a return flow of said blood sample.

8. The transfusion safety device according to claim 1, **characterised in that** the control unit (5) is a handheld device provided with a result reading and interpreting application.

9. The transfusion safety device according to claim 1, **characterised in that** the flow regulator (9) is a magnetic valve with controlled opening, actuated by the control unit (5).

10. The transfusion safety device according to claim 1, **characterised in that** the first blood group checking device (2) incorporates a dynamic QR code (27) that contains information regarding the individual intended to receive the PRBC transfusion to guarantee that the procedure is tracked.

## Patentansprüche

1. Transfusionssicherheitsvorrichtung zum Ausführen einer Prüfung vor einer Transfusion der Blutgruppe aus verpackten roten Blutkörperchen (PRBC), die in einem Beutel (3) enthalten sind, und der Blutgruppe eines Individuums, das eine Transfusion erhält, und zum Bestimmen der Verträglichkeit davon, um die Transfusion zu ermöglichen oder zu verhindern, wobei die Vorrichtung umfasst:
- eine Bluttransfusionseinheit (1), die wiederum umfasst:
- eine Beutelleitung (6) zum Verbinden mit dem Beutel (3), der die PRBC enthält,
- eine Individuumsleitung (7) zum Verbinden mit dem Individuum, das die Transfusion erhält,
- einen Anschluss (8) zum Verbinden der Beutelleitung (6) mit der Empfängerleitung (7) und
- einen Durchflussregler (9), der in der Beutelleitung (6) angeordnet ist, der den Durchgang der Strömung von PRBC, die zwischen der Beutelleitung (6) und der Individuumsleitung (7) zirkuliert, steuert,
- eine erste Blutgruppen-Prüfvorrichtung (2) zum Feststellen Blutgruppe der PRBC, die in dem Beutel (3) enthalten sind,
- eine zweite Blutgruppen-Prüfvorrichtung (4) zum Feststellen der Blutgruppe des Individuums, das die Transfusion erhält, die am Ende einer zweiten Umgehung (17) befindlich ist, die von der Individuumsleitung (7) aus startet, und
- eine Steuereinheit (5) zum gleichzeitigen Auslesen der Ergebnisse, die in der ersten (2) und zweiten (4) Blutgruppen-Prüfvorrichtung erhalten werden, um die Verträglichkeit oder Unverträglichkeit beider Blutgruppen basierend auf dem Auslesen der Ergebnisse festzustellen, die von der ersten (2) und zweiten (4) Blutgruppen-Prüfvorrichtung erhalten werden, und um den Durchflussregler (9) zu betätigen, um den Durchgang der Strömung von PRBC dadurch zu ermöglichen oder zu verhindern, wobei die Vorrichtung **dadurch gekennzeichnet ist, dass**:
- die erste Blutgruppen-Prüfvorrichtung (2) am Ende einer ersten Umgehung (15) befindlich ist, die von der Beutelleitung (6) aus startet und dadurch, dass
- jede von erster (2) und zweiter (4) Blutgruppen-Prüfvorrichtung weiter umfasst:
- eine erste Reaktionskammer (19), worin ein Anti-A-Reagenz für die Agglutinierung von Antikörpern der A-Gruppe aufgenommen ist, die mit einer ersten Auslesekammer (20) verbunden ist,
- eine zweite Reaktionskammer (21), worin ein Anti-B-Reagenz für die Agglutinierung von Antikörpern der B-Gruppe aufgenommen ist, die mit einer zweiten Auslesekammer (22) verbunden ist,
- eine dritte Reaktionskammer (23), die mit einer dritten Auslesekammer (24) für eine Kontrolle verbunden ist, und
- eine Mehrzahl von visuellen Informationselementen (26), die mit jeder der Auslesekammern (20, 22, 24) verbunden ist.

2. Transfusionssicherheitsvorrichtung nach Anspruch 1, **dadurch gekennzeichnet, dass** die Beutelleitung (6) weiter umfasst:
- einen Stempel (10) zum Einführen in den Beutel (3), um die PRBC, die darin enthalten sind, zu extrahieren und
- einen Makroaggregat-Filter (11) zum Rückhalten von Abfall, der in den PRBC vorhanden ist, die in dem Beutel (3) enthalten sind.

3. Transfusionssicherheitsvorrichtung nach Anspruch 1, **dadurch gekennzeichnet, dass** die Individuumsleitung (7) weiter eine Verbindungsstelle (13) zum Einführen in einen Kanal (14) umfasst, der in einer Vene des Empfängers hergestellt ist.

4. Transfusionssicherheitsvorrichtung nach Anspruch 1, **dadurch gekennzeichnet, dass** die Mehrzahl von visuellen Informationselementen (26) eine Mehrzahl von QR-Codes ist.

5. Transfusionssicherheitsvorrichtung nach Anspruch 1, **dadurch gekennzeichnet, dass** die erste (2) und die zweite (4) Blutgruppen-Prüfvorrichtung eine Geometrie haben, die komplementär zueinander ist, um die Verbindung davon vor dem Auslesen der Ergebnisse durch die Steuereinheit (5) zu erleichtern.

6. Transfusionssicherheitsvorrichtung nach Anspruch 1, **dadurch gekennzeichnet, dass** die erste Umgehung (15) ein erstes Rückschlagventil (16) integriert, um einen Rückfluss der PRBC-Probe zu verhindern.

7. Transfusionssicherheitsvorrichtung nach Anspruch 1, **dadurch gekennzeichnet, dass** die zweite Umgehung (17) ein zweites Rückschlagventil (18) integriert, um einen Rückfluss der Blutprobe zu verhindern.

8. Transfusionssicherheitsvorrichtung nach Anspruch 1, **dadurch gekennzeichnet, dass** die Steuereinheit (5) eine tragbare Vorrichtung ist, die mit einer Ergebnisauslese- und -auslegeanwendung bereitgestellt ist.

9. Transfusionssicherheitsvorrichtung nach Anspruch 1, **dadurch gekennzeichnet, dass** der Durchflussregler (9) ein Magnetventil mit gesteuerter Öffnung ist, das durch die Steuereinheit (5) betätigt wird.

10. Transfusionssicherheitsvorrichtung nach Anspruch 1, **dadurch gekennzeichnet, dass** die erste Blutgruppen-Prüfvorrichtung (2) einen dynamischen QR-Code (27) integriert, der Informationen bezüglich des Individuums enthält, das die PRBC-Transfusion erhalten soll, um zu gewährleisten, dass der Vorgang nachverfolgt wird.

## Revendications

1. Dispositif de sécurité transfusionnelle, destiné à effectuer une vérification avant une transfusion du groupe sanguin de concentrés de globules rouges (CGR), contenus dans une poche (3) et du groupe sanguin du sang d'un individu recevant une transfusion et à déterminer la compatibilité de celui-ci pour permettre ou empêcher ladite transfusion, le dispositif comprenant :
- une unité de transfusion sanguine (1) qui comprend à son tour :
- une ligne de poche (6), destinée à être reliée à la poche (3) contenant les CGR,
- une ligne d'individu (7), destinée à être reliée à un individu qui reçoit la transfusion,
- un raccordement (8) pour relier la ligne de poche (6) à la ligne de récipient (7), et
- un régulateur de débit (9) disposé dans la ligne de poche (6), qui contrôle le passage du flux de CGR circulant entre la ligne de poche (6) et la ligne d'individu (7),
- un premier dispositif de vérification de groupe sanguin (2) destiné à établir le groupe sanguin des CGR contenus dans la poche (3),
- un deuxième dispositif de vérification de groupe sanguin (4) destiné à établir le groupe sanguin de l'individu recevant la transfusion, situé à l'extrémité d'une deuxième dérivation (17) qui part de la ligne d'individu (7), et
- une unité de contrôle (5), destinée à lire simultanément les résultats obtenus dans les premier (2) et deuxième (4) dispositifs de vérification de groupe sanguin, à établir la compatibilité ou l'incompatibilité des deux groupes sanguins sur la base de la lecture des résultats obtenus dans les premier (2) et deuxième (4) dispositifs de vérification de groupe sanguin, et à actionner le régulateur de débit (9) afin de permettre ou d'empêcher le passage à travers du flux de CGR, le dispositif étant **caractérisé en ce que** :
- le premier dispositif de vérification de groupe sanguin (2) est situé à l'extrémité d'une première dérivation (15) qui part de la ligne de poche (6), et **en ce que**
- chacun des premier (2) et deuxième (4) dispositifs de vérification de groupe sanguin comprend en outre :
- une première chambre de réaction (19), dans laquelle est logé un réactif anti-A pour l'agglutination d'anticorps du groupe A, reliée à une première chambre de lecture (20),
- une deuxième chambre de réaction (21), dans laquelle est logé un réactif anti-B pour l'agglutination d'anticorps du groupe B, reliée à une deuxième chambre de lecture (22),
- une troisième chambre de réaction (23), reliée à une troisième chambre de lecture (24) pour un contrôle, et
- une pluralité d'éléments d'information visuelle (26) reliés à chacune des chambres de lecture (20, 22, 24).

2. Dispositif de sécurité transfusionnelle selon la revendication 1, **caractérisé en ce que** la ligne de poche (6) comprend en outre :
- un poinçon (10) destiné à être introduit dans la poche (3) pour extraire les CGR contenus à l'intérieur de celle-ci, et
- un filtre de macroagrégats (11) pour retenir les déchets présents dans les CGR contenus dans la poche (3).

3. Dispositif de sécurité transfusionnelle selon la revendication 1, **caractérisé en ce que** la ligne d'individu (7) comprend en outre un point de raccordement (13) destiné à être inséré dans un canal (14) pratiqué dans une veine du récipient.

4. Dispositif de sécurité transfusionnelle selon la revendication 1, **caractérisé en ce que** la pluralité d'éléments d'information visuelle (26) est une pluralité de codes QR.

5. Dispositif de sécurité transfusionnelle selon la revendication 1, **caractérisé en ce que** les premier (2) et deuxième (4) dispositifs de vérification de groupe sanguin ont une géométrie qui est complémentaire entre eux afin de faciliter la liaison de ces derniers avant la lecture de leurs résultats par l'unité de contrôle (5).

6. Dispositif de sécurité transfusionnelle selon la revendication 1, **caractérisé en ce que** la première dérivation (15) incorpore un premier clapet anti-retour (16) pour empêcher un flux de retour dudit échantillon de CGR.

7. Dispositif de sécurité transfusionnelle selon la revendication 1, **caractérisé en ce que** la deuxième dérivation (17) incorpore un deuxième clapet anti-retour (18) pour empêcher un flux de retour dudit échantillon sanguin.

8. Dispositif de sécurité transfusionnelle selon la revendication 1, **caractérisé en ce que** l'unité de contrôle (5) est un dispositif portable pourvu d'une application de lecture et d'interprétation des résultats.

9. Dispositif de sécurité transfusionnelle selon la revendication 1, **caractérisé en ce que** le régulateur de débit (9) est un clapet magnétique à ouverture contrôlée, actionné par l'unité de contrôle (5).

10. Dispositif de sécurité transfusionnelle selon la revendication 1, **caractérisé en ce que** le premier dispositif de vérification de groupe sanguin (2) incorpore un code QR dynamique (27) qui contient des informations concernant l'individu destiné à recevoir la transfusion CGR pour garantir le suivi de la procédure.
